# FASCICULE DE BREVET EUROPEEN

(11) **EP 1 813 230 B1**
(45) Date de publication et mention de la délivrance du brevet: **11.06.2008**
(21) Numéro de dépôt: 07356007.0
(22) Date de dépôt: 23.01.2007
(51) Int. Cl.: A61F 2/46

(54) **Ensemble d'Instrumentation chirurgicale pour poser une prothèse de cheville**
Chirurgisches Instrumentenkit zum Einbringen einer Knöchelprothese.
Chirurgical instumentation kit for placing a dowel prosthesis.

(30) Priorité: 24.01.2006 FR 0600644
(43) Date de publication de la demande: 01.08.2007
(73) Titulaire: TORNIER SA, 38330 Saint Ismier (FR)
(72) Inventeur: Ratron, Yves-Alain, 38000 Grenoble (FR); Cardon, Jean-Emmanuel, 38190 Brighoud (FR); Judet, Thierry, 92410 Ville D'Avray (FR); Colombier, Jean-Alain, 31130 Balma (FR); Bonnin, Michel, 69002 Lyon (FR)
(74) Mandataire: Grand, Guillaume

(56) Documents cités:
- WO-A-00/69373
- WO-A-01/89427
- DE-C1- 10 123 124
- FR-A1- 2 759 900

## Description

La présente invention concerne un ensemble d'instrumention chirurgicale pour poser une prothèse de cheville selon le préambule de la revendication 1. Un tel ensemble d'instrumention chirurgicale est connu du document WO 00/69373 A, par exemple.

Une prothèse de cheville comprend principalement un implant tibial, un implant astragalien et un patin prothétique interposé entre les implants tibial et astragalien. Ce patin est qualifié de « mobile » lorsqu'il est assemblé aux implants tibial et astragalien en étant mobile par rapport à chacun de ces implants, tandis qu'il est qualifié de « fixe » lorsque, tout en étant mobile par rapport à l'implant astragalien, il est rapporté fixement à l'implant tibial.

La mise en place d'une prothèse de cheville, qu'elle soit à plateau mobile ou à plateau fixe, au niveau de la cheville d'un patient nécessite, lors d'une intervention chirurgicale par voie d'abord antérieure, de préparer, notamment par des résections, l'extrémité inférieure du tibia et l'extrémité supérieure de l'astragale du patient, pour y fixer à demeure les implants tibial et astragalien. En pratique, une fois que ces préparations osseuses sont effectuées, le chirurgien a fréquemment recours à des fantômes des implants prothétiques, lui permettant de s'assurer que ces préparations sont convenables et que des dégagements osseux supplémentaires ou des resurfaçages additionnels ne sont pas nécessaires.

Ainsi, le chirurgien met en place, au niveau de la cheville du patient, un fantôme de l'implant astragalien, immobilisé sur l'extrémité réséquée de l'astragale, un fantôme de l'implant tibial, immobilisé en étant plaqué contre l'extrémité réséquée du tibia, et un patin fantôme du patin prothétique, interposé entre les fantômes des implants astragalien et tibial en étant mobile par rapport à chacun de ces fantômes d'implants. En fait, le chirurgien dispose de plusieurs patins fantômes d'épaisseurs respectives différentes, chacun de ces patins fantômes correspondant à un patin prothétique pouvant être ultérieurement implanté, et choisit donc le patin fantôme d'épaisseur la plus appropriée. En effet, en fonction de la morphologie du patient, de la pathologie nécessitant la mise en place de la prothèse et/ou du positionnement relatif des résections effectuées, l'épaisseur du patin à effectivement implanter, c'est-à-dire sa dimension globalement verticale, varie de sorte que le chirurgien dispose, en pratique, d'une série de plusieurs patins prothétiques qui présentent chacun sensiblement les mêmes surfaces de liaison mobile ou fixe avec les implant astragalien et tibial, mais dont les épaisseurs respectives valent 4, 5, 6 et 7 mm par exemple.

Après avoir mis en place les composants fantômes précités, le chirurgien sollicite l'articulation de cheville du patient, notamment suivant des mouvements de flexion-extension. Il contrôle alors, en peropératoire, la qualité des préparations osseuses, ainsi que le comportement dynamique de la cheville munie des composants fantômes, qui est représentatif de celui de la cheville ultérieurement munie des composants prothétiques à implanter.

Malgré les qualités structurelles des prothèses de cheville, le recul clinique montre qu'elles sont assez fréquemment implantées de manière insatisfaisante : lorsqu'il s'agit d'une prothèse à patin fixe, des surcontraintes significatives sont constatées en service entre le patin et l'implant tibial, ce qui conduit, à plus ou moins court terme, à une rupture de la zone de liaison fixe entre cet implant et le patin ou à un endommagement de l'extrémité osseuse du tibia ; lorsqu'il s'agit d'une prothèse à patin mobile, que l'on a tendance à utiliser justement pour contourner l'inconvénient précité des prothèses à patin fixe, on constate que, en service, la surface supérieure du patin prothétique s'étend, plus ou moins largement, en dehors du contour périphérique de la surface inférieure de l'implant tibial, de sorte que les parties de ces surfaces en contact mobile sont plus restreintes que prévu, conduisant à une usure prématurée du patin et/ou de l'implant. Autrement dit, les méthodes de pose actuellement employées ne garantissent pas un positionnement d'implantation relatif efficace entre l'implant tibial et le patin prothétique, que ce dernier soit fixe ou mobile, aucune possibilité de correction fiable n'étant offerte au chirurgien.

Le but de la présente invention est de proposer un ensemble d'intervention chirurgicale pour poser une prothèse de cheville à patin fixe ou mobile, qui permet au chirurgien d'assurer un positionnement d'implantation relatif satisfaisant entre l'implant tibial et le patin de la prothèse de manière à ce que cette prothèse ne subisse pas une dégradation précoce en service.

A cet effet, l'invention a pour objet un ensemble d'instrumentation chirurgicale pour poser une prothèse de cheville, la prothèse incluant un implant tibial, un implant astragalien et un patin prothétique interposé entre les implants tibial et astragalien, cet ensemble d'instrumentation comportant un fantôme tibial de l'implant tibial, un fantôme astragalien de l'implant astragalien et un patin fantôme du patin prothétique, adaptés pour être sollicités conjointement au niveau d'une cheville d'un patient opéré, à des fins de contrôles et d'essais peropératoires,
caractérisé en ce que le fantôme tibial est adapté pour être, à la fois, lié de manière fixe au patin fantôme et déplaçable librement contre une extrémité inférieure du tibia du patient.

Lors de l'intervention chirurgicale visant à implanter une prothèse de cheville, qu'elle soit à patin fixe ou à patin mobile, le chirurgien met en place, au niveau de la cheville du patient préalablement préparée et avant d'implanter définitivement la prothèse, le fantôme tibial, le fantôme astragalien et le patin fantôme de l'ensemble d'instrumentation selon l'invention. Il sollicite ensuite, toujours en peropératoire, l'articulation de cheville du patient, notamment afin de s'assurer que les préparations osseuses sont satisfaisantes et que la taille du patin prothétique à implanter, ainsi qu'éventuellement celles de l'implant tibial et/ou de l'implant astragalien, sont appropriées. Lors de cette sollicitation, aucune liberté de mouvement n'est permise entre le patin fantôme et le fantôme tibial tandis que ce fantôme tibial est librement déplaçable contre l'extrémité inférieure du tibia du patient, ce qui correspond à des capacités dynamiques différentes de celles de l'implant tibial. De la sorte, le patin fantôme, contraint en positionnement par le fantôme astragalien rapporté à l'extrémité supérieure de l'astragale du patient, entraîne de manière correspondante l'implant tibial qui, après cette sollicitation, occupe une position, par rapport au tibia, dans laquelle l'implant tibial à implanter est à fixer à demeure au tibia. En effet, dans cette position, l'implant tibial sera implanté de sorte que le patin prothétique ultérieurement implanté soit placé de manière prédéterminée vis-à-vis de l'implant tibial. Selon que le patin prothétique ultérieurement implanté est soit de type fixe, soit de type mobile, on comprend donc qu'en s'assurant que le fantôme tibial et le patin fantôme soient liés de manière fixe l'un à l'autre soit suivant la même configuration que l'assemblage fixe de l'implant tibial et du patin prothétique ultérieurement implantés, soit de sorte que le patin fantôme est globalement centré sur la surface inférieure du fantôme tibial, le patin prothétique ultérieurement implanté sera, lui aussi, soit assemblé sans surcontrainte à l'implant tibial, soit globalement centré sur la surface inférieure de l'implant tibial, pour autant que cet implant tibial soit ultérieurement implanté sur le tibia dans la position précitée.

En pratique, pour s'assurer d'un positionnement relatif satisfaisant entre, d'une part, le fantôme tibial et le patin fantôme liés de manière fixe l'un à l'autre et, d'autre part, le fantôme astragalien, ce dernier est en avantageusement adapté pour présenter des comportements cinématiques, vis-à-vis du patin fantôme et de l'astragale du patient, au moins en partie analogues à ceux de l'implant astragalien vis-à-vis du patin prothétique et de l'astragale du patient.

De la sorte, lorsque le fantôme astragalien est mis en place et immobilisé à l'extrémité supérieure de l'astragale du patient et que les fantômes de l'ensemble d'instrumentation selon l'invention sont sollicités en peropératoire, le patin fantôme s'articule contre le fantôme astragalien au moins en partie suivant la même cinématique qu'entre l'implant astragalien et le patin prothétique ultérieurement implantés. Autrement dit, la position d'implantation de l'implant tibial, obtenue par le chirurgien comme expliqué ci-dessus, tient compte de la position d'implantation de l'implant astragalien de la prothèse.

Avantageusement, les surfaces articulaires du fantôme astragalien et du patin fantôme, adaptées pour coopérer l'une avec l'autre, sont conjuguées l'une à l'autre en coupe horizontale. De cette façon, aucun débattement médio-latéral n'est permis entre le patin fantôme et le fantôme astragalien lors de la sollicitation peropératoire des composants fantômes au niveau de la cheville du patient, ce qui évite de perturber le positionnement médio-latéral relatif entre l'extrémité inférieure du tibia et le sous-ensemble constitué du patin fantôme et du fantôme tibial fixés l'un à l'autre.

D'autres caractéristiques avantageuses de cet ensemble d'instrumentation chirurgicale, prises isolément ou suivant toutes les combinaisons techniquement possibles, sont énoncées aux revendications dépendantes 4 à 13.

L'invention sera mieux comprise à la lecture de la description qui va suivre, donnée uniquement à titre d'exemple et faite en se référant aux dessins sur lesquels :
- les figures 1 à 3 illustrent une prothèse de cheville à patin mobile, la figure 1 correspondant à une vue éclatée en élévation selon une direction globalement antéro-postérieure, tandis que la figure 2 correspond à une vue éclatée en élévation selon la flèche II indiquée à la figure 1 et que la figure 3 est une coupe éclatée selon la ligne III-III de la figure 2 ;
- les figures 4 et 5 sont des coupes schématiques sagittales du tibia et de l'astragale de la cheville d'un patient, en train d'être préparés en vue de poser la prothèse des figures 1 à 3 ;
- la figure 6 est une vue en perspective de la cheville des figures 4 et 5, une fois que toutes les préparations osseuses ont été effectuées ;
- la figure 7 est une vue éclatée en perspective d'un ensemble d'instrumentation chirurgicale selon l'invention, destiné à permettre la pose de la prothèse des figures 1 à 3 ;
- la figure 8 est une coupe partielle selon le plan VIII de la figure 7 ;
- la figure 9 est une vue schématique en élévation frontale de la cheville du patient dans son état de la figure 6, au niveau de laquelle est mis en place l'ensemble de la figure 7 ;
- les figures 10 et 11 sont respectivement des coupes schématiques selon les lignes X-X et XI-XI de la figure 9 ;
- les figures 12 et 13 sont des vues en perspective d'un ancillaire appartenant à l'ensemble d'instrumentation selon l'invention, cet ancillaire étant représenté seul à la figure 12 et étant associé à un composant de la prothèse des figures 1 à 3 à la figure 13 ; et
- la figure 14 est une vue analogue à la figure 11, illustrant la manipulation de l'ancillaire de la figure 13.

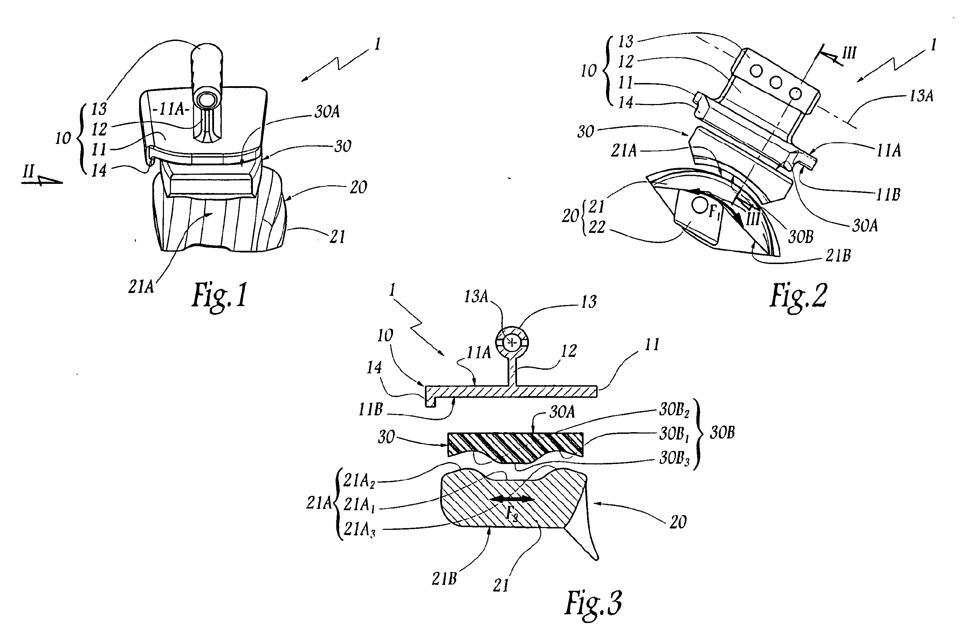

Sur les figures 1 à 3 est représenté un exemple d'une prothèse de cheville dite « à patin mobile ». Cette prothèse comporte trois composants distincts à implanter au niveau de l'articulation d'une cheville droite d'un être humain, à savoir un implant tibial 10, un implant astragalien 20 et un patin prothétique 30. Par commodité, la suite de la description est orientée par rapport aux os d'une cheville dans leur position anatomique, c'est-à-dire que les termes « postérieur » ou « arrière », « antérieur » ou « avant », « droit », « gauche », « supérieur », « inférieur », etc. s'entendent par rapport à la cheville d'un patient se tenant debout sur une surface sensiblement horizontale. De même, le terme « sagittal » correspond à une direction dans le sens antério-postérieur, verticalement sur la ligne médiane de la cheville, tandis que le terme « médial » correspond à une direction sensiblement perpendiculaire au plan sagittal de la cheville, dirigée vers l'autre cheville du patient, le terme « latéral » correspondant à une direction de sens opposé.

L'implant tibial 10 comporte une plaque 11 à fixer à l'extrémité inférieure du tibia droit du patient, après une préparation convenable de cette extrémité. A cet effet, la plaque 11 est, sur son côté supérieur 11A, venue de matière, par une aile sagittale 12, avec un plot creux d'ancrage osseux 13 présentant une forme extérieure globalement cylindrique à base circulaire d'axe 13A s'étendant suivant une direction globalement antéro-postérieure. D'autres moyens d'ancrage osseux de la plaque 11 peuvent être envisagés du moment qu'ils immobilisent efficacement l'implant tibial à l'extrémité inférieure du tibia. Sur son côté médial, la plaque 11 est munie d'un rebord 14 s'étendant vers le bas dans un plan globalement vertical et à même de prendre appui, au moins partiellement, contre la malléole interne du tibia, par l'intérieur de cette malléole. Sur son côté inférieur, la plaque 11 délimite une surface plane 11B destinée à former un appui glissant pour la surface supérieure 30A plane du patin 30.

L'implant astragalien 20 comporte un bloc principal 21 à fixer à l'extrémité supérieure de l'astragale droit du patient, par un plot creux 22 ou tout autre moyen convenable, s'étendant vers le bas depuis le côté inférieur 21B du bloc 21. Sur son côté supérieur, le bloc 21 délimite une surface articulaire 21A destinée à coopérer avec une surface articulaire conjuguée 30B délimitée sur le côté inférieur du patin 30. En coupe sagittale, la surface 21A présente un profil arqué de concavité tournée vers le bas, comme visible à la figure 2. Les surfaces articulaires 21A et 30B sont adaptées pour glisser l'une contre l'autre le long de ce profil incurvé, comme indiqué par la flèche F₁ à la figure 2. En coupe frontale, comme à la figure 3, la surface 21A délimite une zone centrale convexe 21A₁ d'appui glissant pour une zone concave conjuguée 30B₁ de la surface 30A du patin, ainsi que, de part et d'autre de cette zone 21A₁, des zones concaves médiale 21A₂ et latérale 21A₃ le long desquelles peuvent s'appuyer et glisser des zones concaves conjuguées 30B₁ et 30B₂ de la surface 30B. En pratique, la zone centrale 30B₁ est moins large que la zone centrale 21A₁ de façon à ménager un jeu médio-latéral déterminé entre les zones qui raccordent ces zones centrales et les zones médiales 21A₂, 30B₂ et latérales 21A₃, 30B₃. Le jeu précité permet un débattement médio-latéral entre l'implant 20 et le patin 30, comme indiqué par la flèche F₂ à la figure 3.

La prothèse 1 procure une cinématique très proche de celle de l'articulation naturelle de la cheville puisque le patin 30 est mobile, à la fois, par rapport à l'implant tibial 10, selon un appui glissant plan/plan, et par rapport à l'implant astragalien 20, selon les mouvements F₁ et F₂ qui peuvent se combiner.

On va décrire ci-après une méthode chirurgicale visant à implanter la prothèse de cheville 1 des figures 1 à 3, étant entendu que la prothèse considérée n'est qu'un exemple illustratif non limitatif de la méthode et des instruments chirurgicaux utilisés pour implanter cette prothèse. Autrement dit, la méthode et les instruments détaillés ci-après peuvent être utilisés pour implanter des prothèses de cheville de structures très diverses, par exemple dont les implants tibiaux et/ou astragaliens sont constitués de plusieurs parties assemblées les unes aux autres, de nature métallique, plastique et/ou céramique. De plus, comme expliqué plus loin, la méthode et les instruments selon l'invention peuvent être en outre utilisés pour implanter une prothèse de cheville à patin fixe.

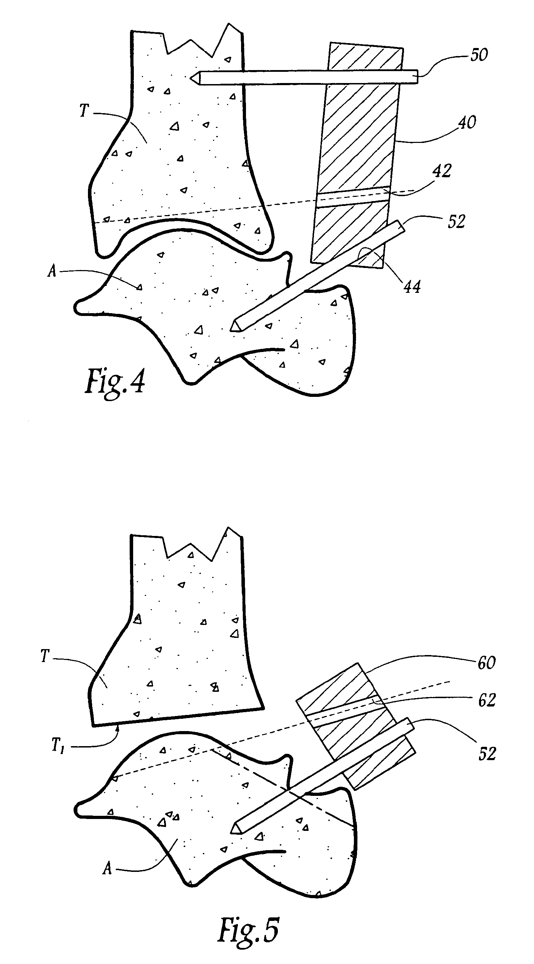

Dans un premier temps, comme représenté aux figures 4 à 6, les os de la cheville droite du patient doivent être préparés. A cet effet, le chirurgien utilise un premier bloc de coupe 40, représenté de manière très schématique à la figure 4, qu'il immobilise sur le tibia T du patient au moyen d'une broche 50 introduite dans le tibia suivant une direction globalement antéro-postérieure. Le bloc 40 délimite une fente 42 de passage d'un moyen de coupe osseuse, dans laquelle le chirurgien introduit et guide, par exemple, une lame de coupe, de manière à réséquer l'extrémité inférieure du tibia T, suivant une ligne de coupe indiquée en pointillés à la figure 4.

Le bloc 40 délimite également un alésage 44 de passage d'une broche 52. Cet alésage 44 est positionné de sorte que la broche 52 soit ancrée dans l'astragale A du patient. Le bloc de coupe 40 est ensuite retiré, tout en laissant en place la broche 52, autour de laquelle un second bloc de coupe 60 est alors enfilé, comme représenté à la figure 5. Ce bloc 60 est destiné à permettre la préparation osseuse de l'astragale A. L'utilisation de la broche 52 permet ainsi de dissocier les préparations tibiale et astragalienne, tout en les rendant dépendantes l'une de l'autre. Le bloc 60 délimite notamment une fente 62 de passage d'un moyen de coupe osseuse, dans lequel le chirurgien introduit et guide une lame de manière à réséquer l'extrémité supérieure de l'astragale A, comme indiqué par les pointillés à la figure 5. Avec un autre bloc de coupe non représenté, lui aussi rapporté sur la broche 52 laissée en position dans l'astragale A, le chirurgien résèque l'extrémité supérieure de l'astragale suivant un autre plan de coupe, indiqué en traits mixtes à la figure 5. L'utilisation de la broche 52 permet ainsi de lier, de façon automatique, la coupe tibiale avec les coupes astragaliennes dans le plan sagittal de la cheville et en rotation. Dans le plan frontal, l'orientation de ces coupes et les épaisseurs de matière osseuse réséquée restent librement choisies par le chirurgien.

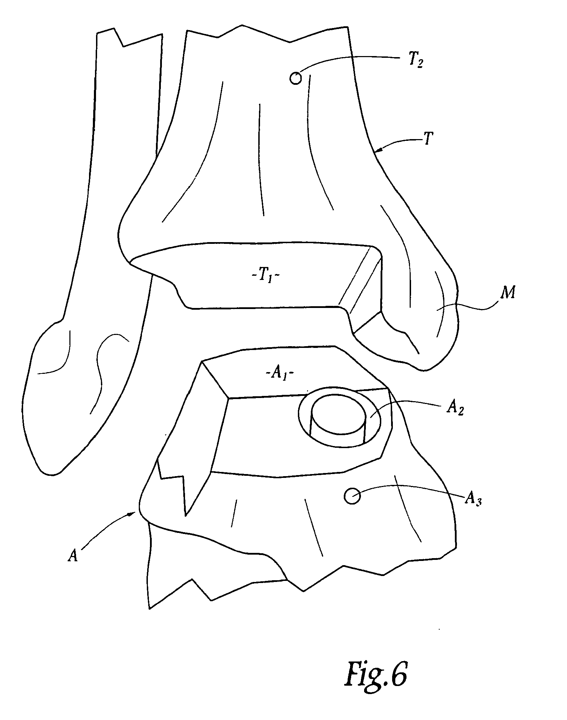

D'autres opérations secondaires de préparation osseuse de l'astragale A sont ensuite réalisées jusqu'à disposer du tibia T et de l'astragale A dans l'état représenté à la figure 6. On retrouve sur cette figure l'extrémité réséquée plane T₁ du tibia T, étant remarqué que cette surface plane de coupe ne débouche pas sur le côté interne de la cheville mais est interrompue de manière à ne pas entamer la malléole interne M du patient. L'extrémité réséquée A₁ de l'astragale A inclut plusieurs plans de coupe, à savoir les deux plans principaux indiqués à la figure 5, ainsi qu'un chanfrein externe et un chanfrein interne (non visible). Dans cette surface réséquée A₁, un évidement tubulaire A₂ est creusé suivant une direction globalement verticale, par exemple au moyen d'une fraise en forme de cloche. De plus, les trous laissés par les broches 50 et 52 sont respectivement référencés T₂ et A₃. En variante non représentée, la préparation des extrémités du tibia et de l'astragale inclut des étapes de resurfaçage de ces extrémités si besoin.

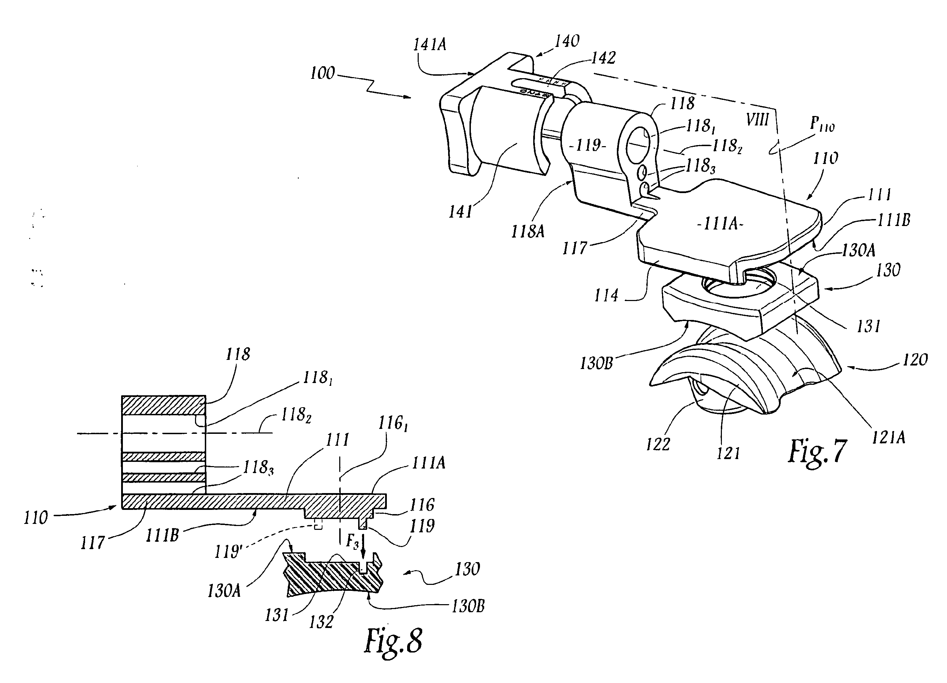

Pour s'assurer que les diverses préparations osseuses du tibia T et de l'astragale A ont été convenablement réalisées en vue d'implanter la prothèse 1, le chirurgien utilise un ensemble d'instrumentation 100 représenté seul aux figures 7 et 8. Cet ensemble comporte trois composants fantômes distincts, à savoir un fantôme tibial 110, un fantôme astragalien 120 et un patin fantôme 130.

Le composant 110 est un fantôme de l'implant tibial 10. Il comporte une plaque principale 111 délimitant une surface supérieure plane 111A qui présente un contour périphérique géométriquement analogue à celui de la plaque 11 de l'implant 10 et qui est munie, sur son côté médial, d'un rebord 114 analogue au rebord 14 de l'implant 10. Sur son côté inférieur, la plaque 111 présente une surface plane 111B d'où s'étend vers le bas, dans la zone centrale de cette surface, un renflement 116 en forme de disque, venu de matière avec le reste de la plaque. L'axe central 116₁ de ce disque s'étend de façon perpendiculaire à la plaque 111, c'est-à-dire suivant une direction globalement verticale, et est situé sensiblement dans le plan vertical médian P₁₁₀ de cette plaque, qui correspond au plan VIII indiqué à la figure 7. Sur son côté antérieur, la plaque 111 est prolongée vers l'avant par une patte horizontale 117 d'où s'étend verticalement vers le haut un aileron 118. Trois alésages traversent cet aileron suivant une direction antéro-postérieure, à savoir un alésage supérieur 118₁ d'axe longitudinal 118₂, et deux alésages inférieurs 118₃ de diamètre inférieur à celui de l'alésage 118₁. L'extrémité supérieure de l'aileron 118 présente une surface extérieure 119 globalement cylindrique à base circulaire, centrée autour de l'axe 118₂. L'axe 118₂ et les axes longitudinaux respectifs des alésages inférieurs 118₃ sont co-planaires et situés sensiblement dans le plan P₁₁₀.

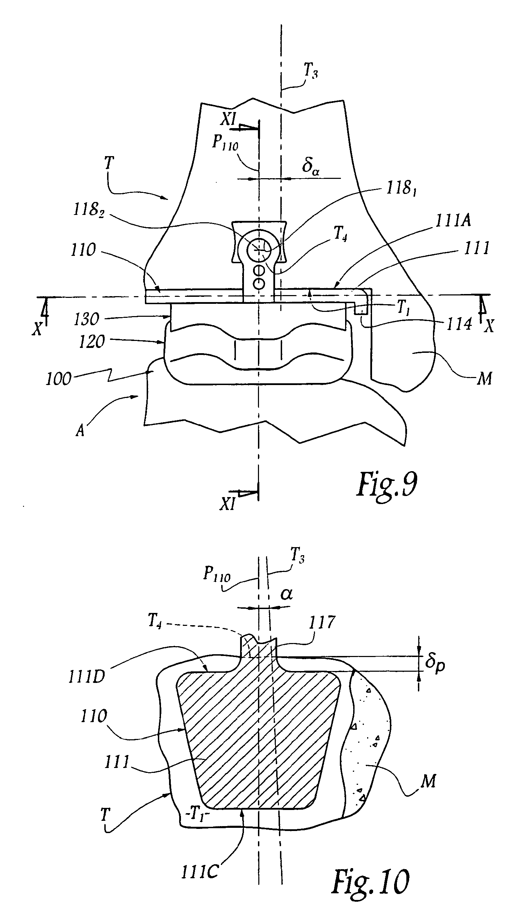

Le composant 130 est un fantôme du patin prothétique 30. Il présente, sur son côté supérieur, une surface plane 130A dans la zone centrale de laquelle est ménagée, vers le bas, une cavité cylindrique à base circulaire 131. Cette cavité 131 est complémentaire du renflement discal 116 du fantôme tibial 110 de sorte que, lorsque le patin fantôme et le fantôme tibial sont assemblés l'un à l'autre comme à la figure 9, le renflement 116 est logé dans la cavité 131. Pour que cet assemblage fantôme tibial/ patin fantôme soit fixe, notamment en rotation autour de l'axe 116₂, un téton excentré 119 s'étend en saillie vers le bas du renflement 116, pour être reçu dans un logement complémentaire 132 creusé dans le patin fantôme 130, dans le fond de la cavité 131, comme indiqué par la flèche F₃ à la figure 8. La coopération du téton 119 et de son logement 132 vise également à indexer angulairement le fantôme tibial 110 et le patin fantôme 130 de sorte que les plans verticaux médians de ces composants soient sensiblement confondus suivant le plan P₁₁₀, comme représenté à la figure 9.

Sur son côté inférieur, le patin fantôme 130 délimite une surface articulaire 130B analogue à la surface articulaire inférieure 30B du patin prothétique 30. De manière conjuguée, un bloc 121 du composant 120, qui est un fantôme de l'implant astragalien 20, délimite, sur son côté supérieur, une surface articulaire 121A analogue à la surface articulaire 21A du bloc 21 de l'implant 20. De la sorte, lorsque le patin fantôme 130 et le fantôme astragalien 120 coopèrent l'un avec l'autre comme à la figure 9, les surfaces 130B et 121A sont articulées l'une contre l'autre selon des comportements cinématiques identiques à ceux correspondant à la coopération des surfaces articulaires 30B et 21A du patin prothétique 30 et de l'implant astragalien 20. Toutefois, pour des raisons développées plus loin, aucun débattement médio-latéral, du type de celui associé à la flèche F₂ de la figure 3, n'est de préférence permis entre le patin fantôme et le fantôme astragalien. Pour ce faire, les surfaces articulaires 121A et 130B sont prévues conjuguées l'une à l'autre en coupe horizontale, à des jeux fonctionnels près. De cette façon, seul un mouvement global de basculement autour d'un axe médio-latéral est autorisé entre les composants fantômes 120 et 130, ce mouvement étant analogue à celui associé à la flèche F₁ de la figure 2.

Sur son côté inférieur, le bloc 121 du fantôme astragalien 120 est muni d'un plot creux 122 d'ancrage osseux, analogue au plot 22 de l'implant astragalien 20.

L'utilisation de l'ensemble d'instrumentation 100 est la suivante :

Le chirurgien met d'abord en place le fantôme astragalien 120, en immobilisant son plot 122 dans l'évidement astragalien A₂. Le fantôme astragalien occupe alors, vis-à-vis de l'astragale A et du tibia T, la même position que celle qu'occupera ultérieurement l'implant astragalien 20 à implanter.

Le chirurgien assemble ensuite le fantôme tibial 110 et le patin fantôme 130, en logeant le renflement 116 dans la cavité 131, de manière à former un sous-ensemble fantôme d'un seul tenant, aucune liberté de mouvement n'étant permise entre le fantôme tibial et le patin fantôme. Ce sous-ensemble fantôme est mis en place au niveau de la cheville du patient, en étant introduit entre l'extrémité réséquée plane T₁ du tibia T et la surface articulaire supérieure 120B du fantôme astragalien 120.

Le chirurgien sollicite ensuite l'articulation de cheville du patient, notamment suivant des mouvements de flexion-extension. Le fantôme astragalien 120 demeure fixement à l'extrémité supérieure de l'astragale A tandis que le sous-ensemble constitué du fantôme tibial 110 et du patin fantôme 130 est, tout en étant plaqué contre l'extrémité réséquée T₁ du tibia, librement entraîné en déplacement vis-à-vis du fantôme astragalien, par coopération des surfaces articulaires 130B et 121A, c'est-à-dire suivant des mouvements relatifs analogues aux mouvements F₁ et F₂, de préférence analogues au mouvement F₁ uniquement, décrits précédemment en regard des figures 2 et 3. Le sous-ensemble fantôme précité se positionne alors, vis-à-vis du fantôme astragalien, de manière automatique de façon à ce que les débattements admissibles entre les surfaces 130B et 121A soient maximaux, en vue de garantir des débattements de même ordre pour les composants prothétiques ultérieurement implantés à la place des composants fantômes. La capacité du sous-ensemble fantôme à se positionner de la sorte est liée à la liberté de mouvement autorisée entre le fantôme tibial 110 et le tibia T, par l'appui glissant plan entre la surface supérieure 111A de la plaque 111 de ce fantôme et l'extrémité inférieure plane réséquée T₁ du tibia. L'ensemble 100, le tibia T et l'astragale A sont alors dans la configuration des figures 9 à 11.

D'un point de vue géométrique, le fantôme tibial 110 est alors déplacé contre l'extrémité tibiale T₁ de sorte que, par exemple, son plan médian P₁₁₀ se trouve incliné par rapport au plan sagittal T₃ du tibia T, sous un angle α non nul en coupe horizontale comme à la figure 10. La zone antérieure du tibia au niveau de laquelle débouche alors l'alésage 118₁ du fantôme tibial est référencée T₄.

En vue frontale antérieure comme à la figure 9, l'inclinaison du plan P₁₁₀ par rapport au plan T₃ se traduit par un décalage médio-latéral δ_{α} au niveau de la face antérieure de la zone tibiale T₄.

On comprend que, pour ne pas perturber le positionnement médio-latéral du sous-ensemble fantôme précité par rapport au tibia T, il peut être préféré qu'aucun débattement médio-latéral significatif ne soit autorisé entre le patin fantôme et le fantôme astragalien, comme indiqué précédemment, sauf à ce que le chirurgien soit attentif à repérer la position du fantôme tibial 110 contre l'extrémité tibiale T₁ après s'être assuré que la surface 130B du patin fantôme occupe une position médiane, selon une direction médio-latérale, vis-à-vis de la surface 121A du fantôme astragalien.

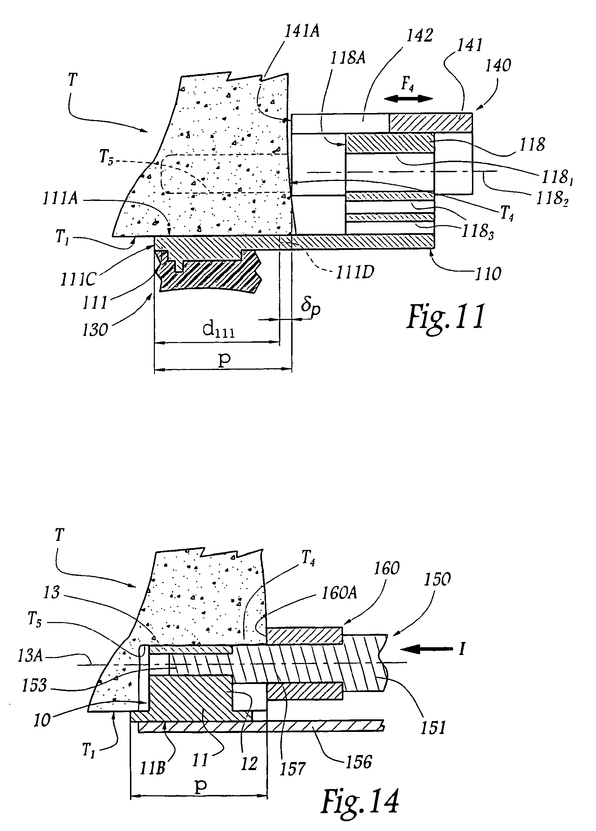

De même, la plaque 111 du fantôme tibial 110 est alors plus ou moins déplacée vers l'arrière vis-à-vis de l'extrémité T₁ du tibia T. Il en résulte que la profondeur p du positionnement de cette plaque, qui correspond, par exemple, à la distance sagittale séparant son bord postérieur 111C de la face antérieure de la zone tibiale T₄, n'est pas nécessairement égale à la dimension sagittale d₁₁₁ de la plaque 111, c'est-à-dire à la distance séparant les bords postérieur 111C et antérieur 111D de cette plaque. Dans l'exemple considéré aux figures, cette profondeur p est supérieure à la dimension d₁₁₁, d'une valeur notée δₚ. Pour quantifier δₚ, autrement dit pour mesurer le positionnement de la plaque 111 en retrait, vers l'arrière, de la face antérieure de la zone tibiale T₄, l'ensemble d'instrumentation 100 inclut avantageusement une douille 140 adaptée pour être rapportée de manière coulissante sur la partie d'extrémité supérieure de l'aileron 118. La douille 140 présente ainsi, par exemple, un corps principal tubulaire 141 creux, dont la paroi intérieure est complémentaire de la surface 119 de l'extrémité supérieure de l'aileron. Ce corps peut ainsi coulisser, suivant l'axe 118₂, le long de cette surface 119, comme indiqué par la flèche F₄, jusqu'à ce que son bord postérieur 141A vienne buter contre la face antérieure de la zone tibiale T₄, comme représenté à la figure 11. Sur son côté supérieur, le corps 141 délimite une fente antéro-postérieure 142 le long de laquelle sont prévues des graduations, par exemple tous les deux millimètres. Par un dimensionnement adéquat de la dimension sagittale entre le bord postérieur 118A de l'aileron 118 et le bord postérieur 111D de la plaque 111, la graduation « 0 » du corps 141 en butée contre la zone tibiale T₄ s'étend sensiblement à l'aplomb du bord 118A de l'aileron lorsque le bord 111D s'étend en affleurement de la face antérieure de la zone tibiale T₄, c'est-à-dire lorsque le retrait δₚ précité est sensiblement nul, tandis que la valeur du retrait vers l'arrière δₚ du bord 111D vis-à-vis de cette face antérieure de la zone T₄ est, le cas échéant, mesurée par la lecture de la graduation s'étendant à l'aplomb du bord 118A de l'aileron 118. Dans l'exemple considéré aux figures, la graduation lue de la sorte est la graduation « 2 », ce qui indique au chirurgien que le retrait vers l'arrière δp du fantôme tibial 110 vaut 2 mm environ.

Après avoir sollicité l'articulation de cheville du patient alors que les composants fantômes 110, 120 et 130 y sont placés comme décrit ci-dessus, le chirurgien dispose d'informations fiables quant à la position précise du fantôme tibial 110 contre l'extrémité réséquée T₁ du tibia T. Cette position est satisfaisante, voire optimale, du point de vue du comportement dynamique ultérieur de la prothèse implantée 1, dans le sens où elle garantit que, tout en prenant en compte la position d'implantation de l'implant astragalien, le patin 30 ultérieurement implanté sera situé en regard de la zone centrale de la surface inférieure 11B de l'implant tibial 10 sous réserve que ce dernier soit implanté dans la position déterminée précitée.

Pour ce faire, alors que les composants fantômes 110, 120 et 130 sont maintenus en place au niveau de la cheville du patient, le chirurgien utilise l'alésage 118₁ du fantôme tibial 110 pour guider le forêt d'une perceuse ou un outil analogue dans la zone tibiale T₄. Le chirurgien creuse ainsi la zone T₄ suivant l'axe 118₂ de l'alésage 118₁, le percement correspondant, indiqué en pointillés à la figure 11 et référencée T₅, étant réalisé avec l'angle α par rapport au plan sagittal T₃ du tibia T et avec le décalage frontal δ_{α} au niveau de la face antérieure du tibia. Le percement T₅ est destiné à recevoir le plot d'ancrage osseux 13 de l'implant tibial 10 si bien que le diamètre de l'alésage 118₁ est avantageusement égal au diamètre extérieur de ce plot 13. Les alésages inférieurs 118₃ de l'aileron 118 sont ensuite avantageusement utilisés pour dégager au moins une partie de la matière osseuse de la zone tibiale T₄, pour permettre ultérieurement le passage par l'avant de l'aile 12 de l'implant 10.

Une fois le percement osseux T₅ réalisé, les composants fantômes 110, 120 et 130 sont dégagés de la cheville pour être remplacés par les composants prothétiques définitifs, à savoir l'implant tibial 10, l'implant astragalien 20 et le patin prothétique 30. L'implant astragalien est mis en lieu et place du fantôme astragalien 120.

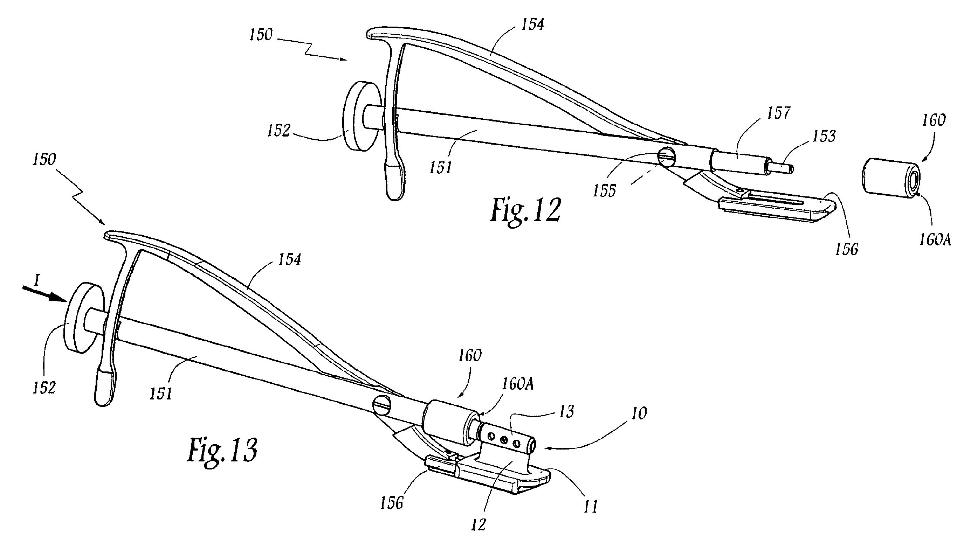

Pour implanter l'implant tibial 10, le chirurgien utilise l'ancillaire 150 représenté aux figures 12 et 13, destiné à permettre l'impaction de l'implant 10 au niveau de l'extrémité épiphysaire du tibia T. A cet effet, cet ancillaire comporte une tige principale d'impaction 151 munie, à son extrémité proximale, d'une tête 152 d'application des efforts d'impaction et, à son extrémité distale, d'un téton 153 à même d'être enfilé dans l'alésage central du plot 13 de l'implant 10, comme représenté aux figures 13 et 14. Pour maintenir fermement l'implant tibial 10 lors de son impaction, l'ancillaire 150 inclut une branche 154 de serrage de l'implant, articulée sur la tige 151 à la façon d'une pince, autour d'un axe transversal 155. A son extrémité distale, cette branche est munie d'un plateau 156 d'appui contre la surface inférieure plane 11B de l'implant 10. Après avoir introduit le téton 153 dans le plot 13, le plateau 156 est amené en appui pressant contre la surface 11B de la plaque 11, par basculement de la branche 154 autour de l'axe 155, de manière à serrer fermement l'implant 10 entre le téton et le plateau.

Lorsque l'implant 10 est maintenu de la sorte par l'ancillaire 150, le chirurgien le positionne au niveau de la zone tibiale T₄ et, en appliquant un effort d'impaction I sur la tête 152, il introduit en force le plot d'ancrage 13 dans le percement tibial T₅. Comme ce percement a été réalisé en tenant compte de l'angle α et du décalage δ_{α}, l'implantation de l'implant 10 est réalisé de sorte que l'axe central 13A de son plot 13 occupe sensiblement la même position, par rapport au tibia T, qu'occupait l'axe 118₂ de l'alésage 118₁ du fantôme tibial 110 après la sollicitation de la cheville dotée des composants fantômes.

L'ancillaire 150 est également adapté pour permettre au chirurgien d'implanter l'implant tibial 10 avec une profondeur sagittale sensiblement identique à la profondeur p qu'occupait le fantôme tibial 110. A cet effet, l'ancillaire est associé à un insert 160 adapté pour être rapporté de manière amovible autour d'une partie distale 157 de la tige 151, étagée par rapport à, à la fois, le téton 153 et le reste de la tige 151. Cet insert se présente sous la forme d'un tube creux dont le diamètre intérieur est sensiblement égal au diamètre extérieur de la partie de tige 157 et dont le bord distal 160A est destiné à venir buter contre la face antérieure du tibia, autour du creusement T₅ recevant le plot d'ancrage tibial 13, lorsque la profondeur p est atteinte. En fait, on comprend que la dimension longitudinale de l'insert 160 est liée à la valeur δₚ mesurée avec le fantôme tibial 110 puisque la mise en butée du bord 160A contre la face antérieure de la zone tibiale T₄ conditionne la profondeur sagittale d'impaction de l'implant 10, comme représenté sur la figure 14. En pratique, le chirurgien dispose d'au moins d'autant d'inserts de longueurs différentes qu'il y a de graduations sur la douille 140, chaque insert correspondant à chaque graduation de manière que la profondeur d'impaction de l'implant tibial 10 corresponde à la profondeur p mesurée grâce à cette douille.

Après l'implantation de l'implant tibial 10, le patin prothétique 30 est rapporté entre cet implant tibial et l'implant astragalien 20, en étant automatiquement centré contre la surface 11B de l'implant tibial 10. On comprend également que si la méthode et l'ensemble d'instrumentation 100 sont utilisés pour implanter une prothèse de cheville à patin fixe, aucune surcontrainte, liée par exemple à un décentrage ou à un cisaillement, n'est alors générée au niveau de la zone de liaison fixe entre l'implant tibial et le patin de cette prothèse : la position d'implantation de l'implant tibial à l'extrémité tibiale est justement déterminée en tenant compte de cette liaison fixe au sein de l'articulation de la cheville, notamment vis-à-vis de l'implant astragalien, puisque le fantôme tibial 110 et le patin fantôme 130 sont sollicités au niveau de la cheville, munie du fantôme astragalien 120, en étant liés fixement l'un à l'autre.

L'utilisation des composants fantômes 110, 120 et 130 présentent d'autres avantages que ceux décrits ci-dessus. Ils permettent notamment d'assurer que les préparations osseuses de l'extrémité inférieure du tibia T et de l'extrémité supérieure de l'astragale A sont satisfaisantes en vue d'implanter la prothèse de cheville. En outre, comme expliqué plus haut, l'utilisation de ces composants fantômes peut permettre de choisir la taille du patin prothétique la plus appropriée à la cheville opérée, le chirurgien disposant alors de plusieurs patins fantômes analogues au patin fantôme 130, présentant des épaisseurs respectives différentes, valant par exemple 4, 5, 6 et 7 mm. De même, plusieurs fantômes tibiaux et/ou astragaliens, présentant des tailles respectives différentes, peuvent être mises à la disposition du chirurgien afin que ce dernier sélectionne la taille des implants ultérieurement implantés la plus appropriée au patient. On comprend alors l'intérêt de pouvoir assembler et immobiliser le patin fantôme 130 d'une taille quelconque avec le fantôme tibial 110 également d'une taille quelconque.

De même, l'ensemble d'instrumentation selon l'invention peut inclure, en plus des composants fantômes utilisés pour l'implantation d'une prothèse de cheville droite telle que la prothèse 1, des composants fantômes destinés à poser une prothèse de cheville gauche, qu'elle soit à patin mobile ou à patin fixe. Dans ce cas, les fantômes tibial et astragalien utilisés pour poser une prothèse de cheville gauche sont obtenus, d'un point de vue géométrique, par symétrie des composants fantômes 110 et 120 décrits ci-dessus par rapport à leur plan vertical médian respectif. Avantageusement, le même patin fantôme 130 peut être utilisé à condition qu'il soit retourné de 180° autour de la verticale avant d'être assemblé au fantôme tibial. En pratique, le fantôme tibial utilisé pour poser une prothèse de cheville gauche présente alors son téton 119' de manière diamétralement opposée au téton 119 du fantôme tibial 110 utilisé pour poser une prothèse de cheville droite, comme indiqué en traits pointillés à la figure 8. Autrement dit, le logement 132 de réception du téton 119 ou du téton 119' est utilisé pour détromper l'assemblage du fantôme tibial avec le patin fantôme selon que la prothèse à poser est une prothèse de cheville droite ou gauche.

Divers aménagements et variantes à l'ensemble d'instrumentation chirurgicale 100 et à la méthode de pose décrits ci-dessus sont par ailleurs envisageables. A titre d'exemples :
- pour renforcer l'immobilisation du renflement 116 dans la cavité 131, la paroi périphérique de ce renflement peut être munie de rainures de clipsage ou d'autres moyens analogues, adaptés pour coopérer par complémentarité de formes avec la paroi périphérique de la cavité 131 ;
- plutôt que de prévoir que le patin fantôme 130 et le fantôme tibial 110 sont deux composants distincts à assembler de manière fixe l'un à l'autre, ces composants fantômes peuvent être réalisés d'une seule et même pièce, du moment que cette dernière définisse, à la fois, une surface plane supérieure analogue à la surface 111A et une surface inférieure articulaire analogue à la surface 130B ;
- plutôt que d'utiliser la douille 140 pour effectuer le repérage antéro-postérieur du fantôme tibial 110, la position en profondeur de ce fantôme peut être marquée sur l'extrémité tibiale, par exemple avec de l'encre biocompatible, étant entendu que le chirurgien utilisera la marque ainsi réalisée pour positionner ensuite l'implant tibial selon une direction antéro-postérieure ;
- le nombre d'alésages antéro-postérieurs traversant l'aileron 118 du fantôme tibial 110 peut être inférieur ou supérieur à trois comme dans l'exemple considéré ;
- l'ensemble d'instrumentation 100 inclut, à titre optionnel, un matériau ou une substance facilitant le glissement du fantôme tibial 110 contre l'extrémité tibiale T₁ lors des sollicitations peropératoires de la cheville ; ces moyens de glissement peuvent prendre des formes très diverses, telles qu'un revêtement glissant prévu sur la surface 111A du fantôme tibial ou qu'un gel lubrifiant biocompatible appliqué sur cette surface ; et/ou
- plutôt que de prévoir des composants fantômes utilisés exclusivement aux seules fins des essais et des contrôles peropératoires, on peut utiliser, dans le cadre de ces essais et contrôles, des composants prothétiques ou, tout au moins, des parties d'éléments prothétiques, qui une fois ces contrôles et essais terminés, sont tout ou en parties utilisés en tant qu'implants à implanter à demeure au niveau de la cheville ; c'est par exemple le cas lorsqu'on cherche à réviser une prothèse de cheville implantée antérieurement, dont l'implant astragalien est alors utilisé en tant que fantôme d'essais et de contrôles, conjointement à un fantôme tibial et à un patin fantôme, en vue d'implanter un nouvel implant tibial et un nouveau patin prothétique.

## Revendications

1. Ensemble d'instrumentation chirurgicale (100) pour poser une prothèse de cheville (1), ladite prothèse incluant un implant tibial (10), un implant astragalien (20) et un patin prothétique (30) interposé entre les implants tibial et astragalien, cet ensemble d'instrumentation comportant un fantôme tibial (110) de l'implant tibial, un fantôme astragalien (120) de l'implant astragalien et un patin fantôme (130) du patin prothétique, adaptés pour être sollicités conjointement au niveau d'une cheville d'un patient opéré, à des fins de contrôles et d'essais peropératoires,
**caractérisé en ce que** le fantôme tibial (110) est adapté pour être, à la fois, lié de manière fixe au patin fantôme (130) et déplaçable librement contre une extrémité inférieure (T₁) du tibia (T) du patient.

2. Ensemble suivant la revendication 1, **caractérisé en ce que** le fantôme astragalien (120) est adapté pour présenter des comportements cinématiques, vis-à-vis du patin fantôme (130) et de l'astragale (A) du patient, au moins en partie analogues à ceux de l'implant astragalien (20) vis-à-vis du patin prothétique (30) et de l'astragale du patient.

3. Ensemble suivant la revendication 2, **caractérisé en ce que** les surfaces articulaires (121A, 130B) du fantôme astragalien (120) et du patin fantôme (130), adaptées pour coopérer l'une avec l'autre, sont conjuguées l'une à l'autre en coupe horizontale.

4. Ensemble suivant l'une quelconque des revendications précédentes, **caractérisé en ce que** le fantôme tibial (110) et le patin fantôme (130) sont des composants distincts à même d'être assemblés l'un à l'autre, respectivement munis de moyens (116, 119, 131, 132) de fixation et d'immobilisation de l'un sur l'autre.

5. Ensemble suivant la revendication 4, **caractérisé en ce que** les moyens de fixation et d'immobilisation respectifs (116, 119, 131, 132) du fantôme tibial (110) et du patin fantôme (130) sont adaptés pour coopérer ensemble par complémentarité de formes.

6. Ensemble suivant l'une des revendications 4 ou 5, **caractérisé en ce qu**'il comporte d'une part, deux fantômes tibiaux, respectivement adaptés pour être utilisés pour poser une prothèse de cheville droite (1) et une prothèse de cheville gauche, et, d'autre part, deux fantômes astragaliens, respectivement adaptés pour être utilisés pour poser une prothèse de cheville droite et une prothèse de cheville gauche, et en ce que le même patin fantôme (130) est adapté pour être indifféremment utilisé conjointement avec soit les fantômes tibial (110) et astragalien (120) associés à la prothèse de cheville droite (1), soit les fantômes tibial et astragalien associés à la prothèse de cheville gauche, les moyens de fixation et d'immobilisation (116, 119, 119', 131, 132) étant adaptés pour détromper l'assemblage de ce patin fantôme avec l'un ou l'autre des deux fantômes tibiaux.

7. Ensemble suivant l'une quelconque des revendications 1 à 3, **caractérisé en ce que** le fantôme tibial et le patin fantôme sont réalisés d'une seule et même pièce.

8. Ensemble suivant l'une quelconque des revendications précédentes, **caractérisé en ce qu**'il inclut des moyens (118, 140) de repérage d'une position donnée qu'occupe le fantôme tibial (110) contre l'extrémité (T₁) du tibia (T) après ladite sollicitation de ce fantôme tibial, du fantôme astragalien (120) et du patin fantôme (130) au niveau de la cheville.

9. Ensemble suivant la revendication 8, **caractérisé en ce que** les moyens de repérage (118, 140) sont portés, éventuellement de manière mobile, par le fantôme tibial (110).

10. Ensemble suivant l'une des revendications 8 ou 9, **caractérisé en ce que** les moyens de repérage comprennent un moyen (118) de guidage d'un outil de perçage ou de coupe du tibia (T), adapté pour appliquer cet outil dans le tibia avec un angle (α) et un décalage (δ_{α}), par rapport à un plan prédéterminé (T₃) du tibia, associés à ladite position donnée, notamment avec un angle horizontal (α) et un décalage médio-latéral (δ_{α}) par rapport au plan sagittal (T₃) du tibia.

11. Ensemble suivant l'une quelconque des revendications 8 à 10, **caractérisé en ce que** les moyens de repérage comprennent un moyen (140) de mesure de la profondeur (p) de ladite position donnée par rapport à une face du tibia (T), notamment la face antérieure du tibia.

12. Ensemble suivant la revendication 11, **caractérisé en ce qu**'il comporte en outre un ancillaire (150) d'impaction de l'implant tibial (10) et un insert (160) adapté pour être rapporté sur l'ancillaire de manière à buter contre ladite face du tibia lorsque l'implant tibial occupe une position, par rapport à l'extrémité (T₁) du tibia (T), dont la profondeur est sensiblement égale à la profondeur (p) de ladite position donnée.

13. Ensemble suivant l'une quelconque des revendications précédentes, **caractérisé en ce qu**'il inclut des moyens de glissement entre l'extrémité inférieure (T₁) du tibia (T) et une surface (111A) du fantôme tibial (110) adaptée pour être appuyée de manière déplaçable contre cette extrémité.

## Claims

1. Surgical instrumentation kit (100) for fitting an ankle prosthesis (1), the said prosthesis including a tibial implant (10), a talar implant (20) and a prosthetic bearing (30) interposed between the tibial and talar implants, this instrumentation kit comprising a tibial phantom (110) of the tibial implant, a talar phantom (120) of the talar implant and a phantom bearing (130) of the prosthetic bearing, which are adapted to be stressed jointly in the region of an ankle of a patient undergoing surgery, for peroperative testing and checking purposes, **characterised in that** the tibial phantom (110) is adapted to be both fixedly connected to the phantom bearing (130) and freely displaceable against a lower end (T₁) of the patient's tibia (T).

2. Kit according to Claim 1, **characterised in that** the talar phantom (120) is adapted to display kinematic characteristics, with respect to the phantom bearing (130) and the patient's talus (A), which are at least partly analogous to those of the talar implant (20) with respect to the prosthetic bearing (30) and the patient's talus.

3. Kit according to Claim 2, **characterised in that** the articular surfaces (121A, 130B) of the talar phantom (120) and of the phantom bearing (130), which surfaces are adapted to cooperate with each other, are complementary to each other in horizontal section.

4. Kit according to any one of the preceding claims, **characterised in that** the tibial phantom (110) and the phantom bearing (130) are distinct components capable of being assembled to each other, respectively provided with means (116, 119, 131, 132) for fixing and immobilising one on the other.

5. Kit according to Claim 4, **characterised in that** the respective fixing and immobilising means (116, 119, 131, 132) of the tibial phantom (110) and of the phantom bearing (130) are adapted to cooperate together by complementarity of shapes.

6. Kit according to one of Claims 4 and 5, **characterised in that** it comprises, on the one hand, two tibial phantoms, respectively adapted to be used for fitting a right ankle prosthesis (1) and a left ankle prosthesis, and, on the other hand, two talar phantoms, respectively adapted to be used for fitting a right ankle prosthesis and a left ankle prosthesis, and **in that** the same phantom bearing (130) is adapted to be used equally well together with either the tibial phantom (110) and talar phantom (120) associated with the right ankle prosthesis (1), or the tibial phantom and talar phantom associated with the left ankle prosthesis, the fixing and immobilising means (116, 119, 119', 131, 132) being adapted to key the assembly of this phantom bearing with either of the two tibial phantoms.

7. Kit according to any one of Claims 1 to 3, **characterised in that** the tibial phantom and the phantom bearing are made as a single part.

8. Kit according to any one of the preceding claims, **characterised in that** it includes means (118, 140) for locating a given position occupied by the tibial phantom (110) against the end (T₁) of the tibia (T) after the said stressing of this tibial phantom, of the talar phantom (120) and of the phantom bearing (130) in the region of the ankle.

9. Kit according to Claim 8, **characterised in that** the locating means (118, 140) are borne, optionally movably, by the tibial phantom (110).

10. Kit according to one of Claims 8 and 9, **characterised in that** the locating means comprise a means (118) for guiding a tool for drilling or cutting the tibia (T), which means (118) is adapted to apply this tool in the tibia with an angle (α) and an offset (δ_{α}), with respect to a predetermined plane (T₃) of the tibia, which are associated with the said given position, in particular with a horizontal angle (α) and a medio-lateral offset (δ_{α}) with respect to the sagittal plane (T₃) of the tibia.

11. Kit according to any one of Claims 8 to 10, **characterised in that** the locating means comprise a means (140) for measuring the depth (p) of the said given position with respect to a face of the tibia (T), in particular the anterior face of the tibia.

12. Kit according to Claim 11, **characterised in that** it further comprises an accessory (150) for impacting the tibial implant (10) and an insert (160) adapted to be fitted on the instrument so as to abut against the said face of the tibia when the tibial implant occupies a position, with respect to the end (T₁) of the tibia (T), the depth of which is substantially equal to the depth (p) of the said given position.

13. Kit according to any one of the preceding claims, **characterised in that** it includes sliding means between the lower end (T₁) of the tibia (T) and a surface (111A) of the tibial phantom (110) which is adapted to be displaceably pressed against this end.

## Patentansprüche

1. Chirurgisches Instrumentenkit (100) zum Einbringen einer Knöchelprothese (1), wobei die Prothese ein Schienbeinimplantat (10), ein Knöchelimplantat (20) und einen zwischen dem Schienbeinimplantat und dem Knöchelimplantat angeordneten Prothesepuffer (30) enthält, wobei das Instrumentenkit einen Schienbeinabdruck (110) des Schienbeinimplantats, einen Knöchelabdruck (120) des Knöchelimplantats und einen Pufferabdruck (130) des Prothesepuffers umfasst, die dafür ausgelegt sind, zu perioperativen Kontroll- und Versuchszwecken gemeinsam in Höhe eines Knöchels eines operierten Patienten verbunden zu werden,
**dadurch gekennzeichnet, dass** der Schienbeinabdruck (110) dazu ausgebildet ist, gleichzeitig auf feste Weise mit dem Pufferabdruck (130) verbunden zu werden und frei zu einem unteren Ende (T₁) des Schienbeins (T) des Patienten beweglich zu sein.

2. Kit nach Anspruch 1, **dadurch gekennzeichnet, dass** der Knöchelabdruck (120) dazu ausgebildet ist, kinematisches Verhalten gegenüber dem Pufferabdruck (130) und dem Knöchel (A) des Patienten auf eine Weise auszuüben, die mindestens zum Teil analog zu der des Knöchelimplantats (20) gegenüber dem Prothesepuffer (30) und dem Knöchel des Patienten ist.

3. Kit nach Anspruch 2, **dadurch gekennzeichnet, dass** die Gelenkflächen (121A, 130B) des Knöchelabdrucks (120) und des Pufferabdrucks (130), welche dazu ausgebildet sind, miteinander zusammenzuwirken, im Horizontalschnitt miteinander verbunden sind.

4. Kit nach einem der vorhergehenden Ansprüche, **dadurch gekennzeichnet, dass** der Schienbeinabdruck (110) und der Pufferabdruck (130) getrennte Bauteile sind, die zugleich miteinander verbunden sind und jeweils mit Mitteln (116, 119, 131, 132) zur Befestigung und Immobilisierung zueinander versehen sind.

5. Kit nach Anspruch 4, **dadurch gekennzeichnet, dass** die Mittel zur jeweiligen Befestigung und Immobilisierung (116, 119, 131, 132) des Schienbeinabdrucks (110) und des Pufferabdrucks (130) dazu ausgebildet sind, durch eine Komplementarität der Formen zusammenzuwirken.

6. Kit nach einem der Ansprüche 4 oder 5, **dadurch gekennzeichnet, dass** es einerseits zwei Schienbeinabdrücke umfasst, die jeweils dazu ausgebildet sind, zum Einbringen einer Prothese für den rechten Knöchel (1) und einer Prothese für den linken Knöchel verwendet zu werden, und andererseits zwei Knöchelprothesen umfasst, die jeweils dafür ausgebildet sind, zum Einbringen einer Prothese für den rechten Knöchel und einer Prothese für den linken Knöchel verwendet zu werden, und **dadurch**, dass derselbe Pufferabdruck (130) dazu ausgelegt ist, zusammen entweder mit dem Schienbeinabdruck (110) und dem Knöchelabdruck (120), die mit der rechten Knöchelprothese (1) verbunden sind, oder mit dem Schienbeinabdruck und dem Knöchelabdruck, die mit der Knöchelprothese für den linken Knöchel verbunden sind, indifferent verwendet zu werden, wobei die Mittel zur Befestigung und Immobilisierung (116, 119, 119', 131, 132) dazu ausgebildet sind, die Anordnung dieses Pufferabdrucks mit dem einen oder anderen Schienbeinabdruck anzubringen.

7. Kit nach einem der Ansprüche 1 bis 3, **dadurch gekennzeichnet, dass** der Schienbeinabdruck und der Pufferabdruck aus einem einzigen und demselben Stück geformt sind.

8. Kit nach einem der vorhergehenden Ansprüche, **dadurch gekennzeichnet, dass** es Mittel (118, 140) zum Kennzeichnen einer gegebenen Position enthält, die der Schienbeinabdruck (110) zu dem Ende (T₁) des Schienbeins (T) nach der Verbindung dieses Schienbeinabdrucks mit dem Knöchelabdruck (120) und dem Pufferabdruck (130) in Höhe des Knöchels einnimmt.

9. Kit nach Anspruch 8, **dadurch gekennzeichnet, dass** die Kennzeichnungsmittel (118, 140), ggf. auf bewegliche Weise, von dem Schienbeinabdruck (110) getragen werden.

10. Kit nach einem der Ansprüche 8 oder 9, **dadurch gekennzeichnet, dass** die Kennzeichnungsmittel ein Mittel (118) zum Führen eines Bohrwerkzeugs oder eines Werkzeugs zum Schneiden des Schienbeins (T) zur Verwendung dieses Werkzeuges am Schienbein mit einem Winkel (α) und einer Verschiebung (δₐ) bezüglich einer vorbestimmten Ebene (T₃) des Schienbeins, die mit der gegebenen Position verbunden ist, insbesondere mit einem Horizontalwinkel (α) und einer medialateralen Verschiebung (δ₁₁) bezüglich der sagittalen Ebene (T₃) des Schienbeins, umfassen.

11. Kit nach einem der Ansprüche 8 bis 10, **dadurch gekennzeichnet, dass** die Kennzeichnungsmittel ein Mittel (140) zum Messen der Tiefe (p) der gegebenen Position bezüglich einer Fläche des Schienbeins (T), insbesondere der Vorderseite des Schienbeins, umfassen.

12. Kit nach Anspruch 11, **dadurch gekennzeichnet, dass** es außerdem einen Anker (150) zum Einbringen des Schienbeinimplantats (10) und einen Einsatz (160) umfasst, der dazu ausgelegt ist, so mit dem Anker verbunden zu werden, dass er an der Seite des Schienbeins anliegt, wenn das Schienbeinimplantat eine Position bezüglich des Endes (T₁) des Schienbeins (T) einnimmt, deren Tiefe im Wesentlichen gleich der Tiefe (f) der gegebenen Position ist.

13. Kit nach einem der vorhergehenden Ansprüche, **dadurch gekennzeichnet, dass** es Gleitmittel zwischen dem unteren Ende (T₁) des Schienbeins (T) und einer Oberfläche (111A) des Schienbeinabdrucks (110) umfasst, die dazu ausgebildet sind, auf bewegliche Weise an diesem Ende anzuliegen.
